# EUROPEAN PATENT SPECIFICATION

(11) **EP 1 891 014 B1**
(45) Date of publication and mention of the grant of the patent: **11.03.2009**
(21) Application number: 06809919.1
(22) Date of filing: 09.05.2006
(51) Int. Cl.: C07D 223/16

(54) **IMPROVED PROCESS FOR CRYSTALLIZATION OF BENAZEPRIL HYDROCHLORIDE**
VERBESSERTES VERFAHREN ZUR KRISTALLISIERUNG VON BENAZEPRILHYDROCHLORID
PROCESSUS AMELIORE DE CRISTALLISATION DE CHLORHYDRATE DE BENAZEPRIL

(30) Priority: 12.05.2005 IN MU05782005
(43) Date of publication of application: 27.02.2008
(73) Proprietor: Lupin Ltd., Mumbai, Maharashtra 400 098 (IN)
(72) Inventor: SINGH, Girij Pal, Lupin Limited (Research Park), Pune 411 042, Maharashtra (IN); DHAKE, Vilas Nathu, Lupin Limited (Research Park), Pune 411 042, Maharashtra (IN); CHILLARA, Sureshbabu Venkata Sesha, Lupin Limited, Pune 411 042, Maharashtra (IN); GODBOLE, Himanshu Madhav, Lupin Limited, Pune 411 042, Maharashtra (IN)
(74) Representative: Watson, Robert James
(86) International application number: PCT/IN2006/000161
(87) International publication number: WO 2007/015263

(56) References cited:
- WO-A-20/04013105
- WO-A2-02/076375
- WO-A2-20/05009972
- US-A- 4 410 520
- BEYER S K ET AL: "NOTIZ ZUR SYNTHESE EINES OPTISCH AKTIVES ACE-HEMMERS MIT AMINO-OXO-BENZAZEPIN-1-ALKANSAEURE-STRUKTU R MITTELS ENANTIOKONVERGIERENDER, KRISTALLISATIONSINDUZIERTER RACEMAT-TREMUNG" HELVETICA CHIMICA ACTA, VERLAG HELVETICA CHIMICA ACTA. BASEL, CH, vol. 71, 1988, pages 337-343, XP002207338 ISSN: 0018-019X

## Description

### FIELD OF THE INVENTION

The present invention relates to an improved process for crystallization of Benazepril hydrochloride.

### BACKGROUND OF THE INVENTION

Benazepril (CAS REGISTRY No. 86541-75-5) first disclosed in US 4,410,520 is one of the well-known ACE inhibitors and is used for the treatment of hypertension.

Chemically, Benazepril, is (3S)-1-(carboxymethyl-[[(1(S)-1-(ethoxycarbonyl)-3-phenylpropyl]amino]-2,3,4,5-tetrahydro-1H-[1]benzazepine-2-one.

Benazepril is administered orally in the form of hydrochloride salt (CAS REGISTRY No. 86541-74-4) represented by formula (I).

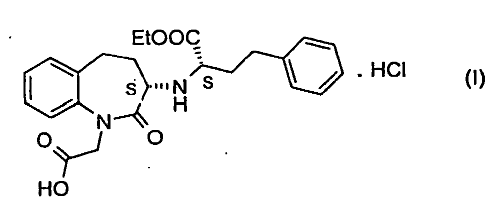

The preparation of benazepril disclosed in US 4,410,520, J. Med. Chem. 1985, 28, 1511-1516, and Helvetica Chimica Acta (1988) 71, 337-342, as given in scheme 1, involves reductive amination of ethyl 2-oxo-4-phenyl butyrate (IV) with sodium salt of (3S)-3-amino-1-carboxymethyl-2,3,4,5-tetrahydro-1 H-benzazepin-2-one (III).

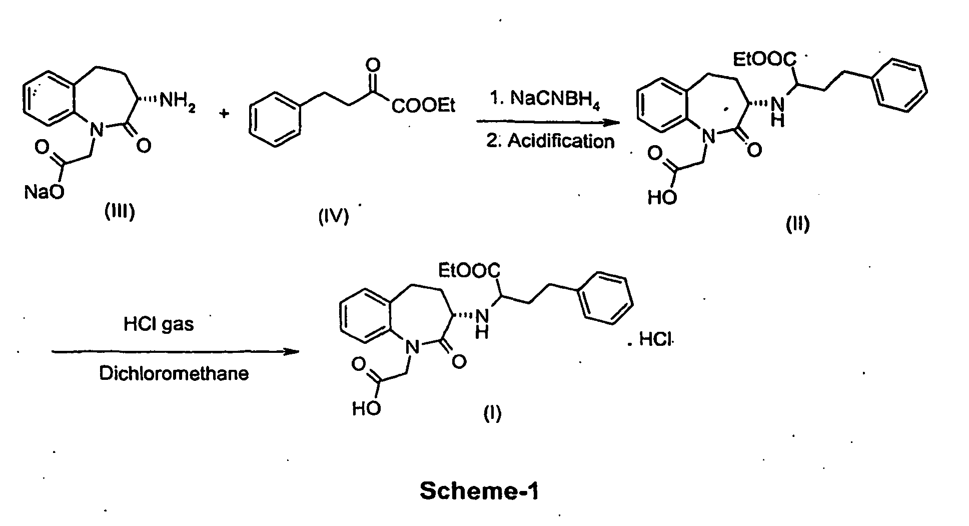

In example 12 of US 4,410,520, the crude benazepril (II) obtained in a diastereomeric ratio of SS: SR=70:30 was dissolved in dichloromethane and treated with HCl gas to obtain benazepril hydrochloride. The benazepril hydrochloride of formula (I) obtained as a foam was crystallized from methyl ethyl ketone to obtain in a SS: SR=95:5 diastereomeric ratio. Benazepril hydrochloride was further purified by recrystallization from a mixture of 3-pentanone/methanol (10:1), melting point: 188-190 °C.

Alternatively, in example 27 of US 4,410,520, benazepril hydrochloride was purified by refluxing in chloroform, filtering, and washing first with chloroform and then with diethyl ether. The melting point of benazepril hydrochloride obtained as per this example is 184-186 °C.

An alternative process disclosed in US 4,785,089 involves nucleophilic substitution of (3S)-3-amino-1-t-butoxycarbonylmethyl-2,3,4,5-tetrahydro-1H-benzazepine-2-one (V), using the chiral substrate ethyl (2R)-2-(4-nitrobenzenesulfonyl)-4-phenyl butyrate (VI) in presence of N-methylmorpholine (scheme 2). The benazepril t-butyl ester (IIa) obtained in a diastereomeric ratio of SS: SR=96:4 was hydrolyzed to benazepril (II) and converted to hydrochloride salt by treating with HCl gas in ethyl acetate. The crystalline suspension of benazepril hydrochloride in ethyl acetate was diluted with acetone and filtered to obtain in a diastereomeric ratio of SS: SR=99.1:0.9. Further purification by refluxing in ethyl acetate afforded benazepril hydrochloride in a diastereomeric ratio of SS: SR=99.7:0.3, melting point of 181 °C.

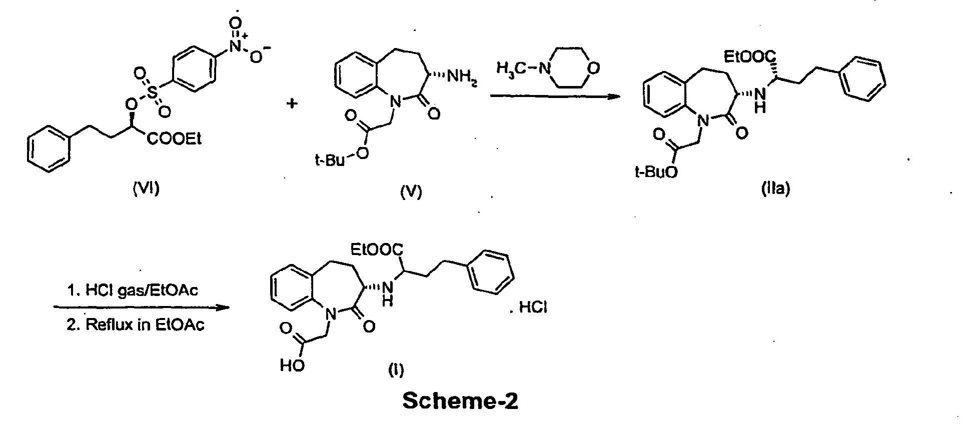

The above documents do not disclose the crystalline form of benazepril hydrochloride obtained by following the purification processes disclosed in the examples.

The Merck Index., 12^{th} edition reports benazepril hydrochloride crystals obtained from 3-pentanone+methanol (10:1), melting point 188-190 °C

The crystallization methods taught in the prior art does not consistently produce a constant diastereomeric composition of SS:SR diastereomer. This is evident from the variation in the melting points of the benazepril hydrochloride reported in three different working examples, which varies between 181 to 190°C.

The variation in diastereomeric composition of a pharmaceutical substance is not desirable as it would affect its efficacy. Hence there is a need for a crystallization process that consistently produce a constant diastereomeric composition of SS diastereomer in greater than 99.8%.

Coming to the crystalline form, it is well known in the art that the solid form of a pharmaceutical substance affect the dissolution rate, solubility and bioavailability. The solid form may be controlled by process employed for the manufacture of the pharmaceutical substance. In particular the process of purification of the solid substance by crystallization is used to control the solid form (Organic Process Research & Development, 2003, 7, 958-1027).

It has been found that the crystalline form of benazepril hydrochloride obtained from processes of prior art documents is designated as crystalline Form A as evident from the following documents.

In a monograph published by Al-badar et al in Profiles of Drug Substances, Excipients, and Related Methodology, Vol. 31, 2004, p117-161; benazepril hydrochloride prepared by the process disclosed in US 4,410,520, and J. Med. Chem. 1985, 28, 1511-1516, has been characterized by powder X-ray diffraction pattern having 2θ peaks at 6.6, 9.9, 11.9, 13.7, 14.0, 14.9, 15.3, 16.4, 17.3, 18.9, 19.6, 20.2, 20.9, 21.5, 22.2, 25.2, 25.5, 26.4, 26.6, 27.1, 27.9, 29.8, 30.4, 31.0, 32.6, 33.3, 33.8, 34.4, 35.5, 38.2, 39.9, 43.9, 48.9.

The major peaks are at 6.6, 9.9, 11.9, 13.7, 14.9, 16.4, 17.3, 18.9, 19.6, 20.2, 20.9, 21.5, 25.2, 25.5, 26.4, 26.6, 27.9, 31.0, and 32.6.

WO 2004/013105 A1 also discloses that by following the processes of the prior art mentioned above, crystalline benazepril hydrochloride is isolated in a form designated as Form A having a powder X-ray diffraction pattern with 2θ values at 6.7, 10.1, 12.0, 13.8, 15.1, 16.4, 17.4, 19.0, 19.6, 20.2, 20.9, 21.0, 25.3, 25.5, 26.4, 26.6, 27.6, 28.0, 31.0, 32.7.

WO 2004/013105 A1 discloses that benazepril hydrochloride Form A may be prepared from a concentrated solution of the benazepril hydrochloride in a solvent selected from C₁-C₁₀ alcohol, N,N-dimethylformamide, N-methylpyrrolidone by adding an anti-solvent selected from C₄-C₁₂ alkane or C₁-C₁₀ acetate, preferably, hexane or ethyl acetate.

WO 2004/013105 A1 in Example 5 describes a process of making crystalline form A of benazepril hydrochloride by passing HCl gas into a solution of benazepril free base in diethyl ether and filtering the resulting suspension.

Similarly, in Example 6, the benazepril hydrochloride was dissolved in water free ethanol and the resulting solution was added to heptane at 20° C to obtain the crystalline Form A.

Further, WO 2004/013105 A1, mentions a list of solvents and anti-solvents that can be used to make benazepril hydrochloride crystalline Form A. However, there is no enabling disclosure and the document is silent on the diastereomeric purity of the crystalline form A obtainable by the process disclosed.

The processes of crystallization and/or recrystallization disclosed in the prior art do not consistently produce benazepril hydrochloride with constant diasteromeric content as evident from the variation in the melting point of the crystalline benazepril hydrochloride obtained from crystallization from various solvents.

The present inventors have now found that use of a mixture of ethanol and diisopropyl ether as a recrystallizing solvent system, benazepril hydrochloride can be consistently obtained in least 99.8% diasteromeric purity.

### OBJECT OF THE INVENTION

Thus it is an object of the present invention to provide a process consistently provides benazepril hydrochloride with SS diastereomeric content of at least 99.8% and above and simultaneously provides benazepril hydrochloride in crystalline form A.

A further object of the present invention is to provide a process that consistently produces benazepril hydrochloride in crystalline form A with constant diastereomeric content of 99.8% and above by use of a solvent system for crystalization of

### SUMMARY OF THE INVENTION

An improved process for the crystallization of benazepril hydrochloride to obtain in at least 99.8% diastereomeric purity, which comprises of (a) making a concentrated solution of benazepril hydrochloride in ethanol (b) adding the resulting solution to diisopropyl ether.

### DETAILED DESCRIPTION OF INVENTION

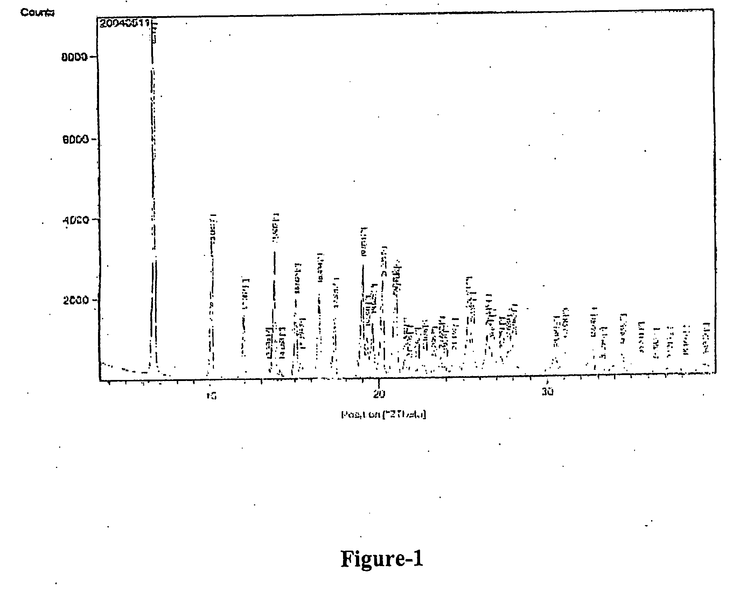

The present inventors have found that use of a mixture of ethanol and diisopropyl ether as a recrystallizing solvent system, benazepril hydrochloride can be consistently obtained in least 99.8% diasteromeric purity and in a solid form having a characteristic X-ray powder diffraction pattern given in Figure 1 with 2θ values at 6.7, 10.1, 12.0, 13.8, 15.1, 16.4, 17.4, 19.0, 19.6, 20.2, 20.9, 21.0, 25.3, 25.5, 26.4, 26.6, 27.6, 28.0, 31.0, 32.7.

The ethanol used is preferably water-free., i.e, absolute ethanol.

Accordingly, a concentrated solution of benazepril hydrochloride in absolute ethanol is added to diisopropyl ether with stirring at a temperature between 20-30 °C. The solid obtained was filtered and dried under reduced pressure.

The solid form obtained by the present process is identical to the solid form disclosed in Profiles of Drug Substances, Excipients, and Related Methodology, Vol. 31, 2004, p117-161and WO 2004/013105 A1.

The process of crystallization of present invention is useful to purify the crude benazepril monohydrochloride obtained by any of the processes disclosed in the prior art document.

The invention is further illustrated by the following non-limiting example.

### EXAMPLE

Benazepril hydrochloride (18.8g) was dissolved in absolute ethanol (94ml). Charcoal (0.75g) was added, stirred, and filtered. Filtrate was concentrated to about 1/3 of its original volume at 40° C under reduced pressure. The concentrated solution was added to diisopropyl ether (263 ml) with stirring at about 20-30° C. The solid separated was filtered off and dried under reduced pressure at 45-50 °C for 7h. The benazepril hydrochloride obtained was found to be having a diastereomeric ratio of SS: SR =99.8: 0.2; by HPLC.
Melting point 187-189° C.

Benazepril hydrochloride obtained by the above process is further characterized by X-ray powder diffraction pattern given in Figure 1 with 2θ values at 6.7, 10.1, 12.0, 13.8, 15.1, 16.4, 17.4, 19.0, 19.6, 20.2, 20.9, 21.0, 25.3, 25.5, 26.4, 26.6, 27.6, 28.0, 31.0, 32.7.

## Claims

1. An improved process for the crystallization of benazepril hydrochloride to obtain in at least 99.8% diastereomeric purity, which comprises of (a) making a concentrated solution of benazepril hydrochloride in ethanol (b) adding the resulting solution to diisopropyl ether.

2. A process according to claim 1 wherein the benazepril hydrochloride is obtained in a crystalline form having a characteristic X-ray powder diffraction pattern with 2θ values at 6.7, 10.1, 12.0, 13.8, 15.1, 16.4, 17.4, 19.0, 19.6, 20.2, 20.9, 21.0, 25.3, 25.5, 26.4, 26.6, 27.6, 28.0, 31.0, 32.7.

3. A process according to claim 1, wherein the ethanol is absolute ethanol. ,

4. A process according to claim 1, wherein the concentrated solution of benazepril hydrochloride in ethanol is added to diisopropyl ether at temperature about 10-35° C, preferably 20-30° C.

5. A process according to claim 1, wherein the crystalline form of benazepril hydrochloride is isolated by filtration and dried under reduced pressure at 45-50° C.

## Patentansprüche

1. Verbessertes Verfahren zur Kristallisierung von Benazeprilhydrochlorid zum Erhalt in zumindest 99,8%iger diastereomerer Reinheit, umfassend (a) die Herstellung einer konzentrierten Lösung aus Benazeprilhydrochlorid in Ethanol und (b) die Zugabe der resultierenden Lösung zu Diisopropylether.

2. Verfahren nach Anspruch 1, worin das Benazeprilhydrochlorid in einer kristallinen Form erhalten wird, die ein charakteristisches Röntgenstrahlen-Pulverdiffraktionsmuster mit 2θ-Werten bei 6,7, 10,1, 12,0, 13,8, 15,1, 16,4, 17,4, 19,0, 19,6, 20,2, 20,9, 21,0, 25,3, 25,5, 26,4, 26,6, 27,6, 28,0, 31,0, 32,7 aufweist.

3. Verfahren nach Anspruch 1, worin das Ethanol absolutes Ethanol ist.

4. Verfahren nach Anspruch 1, worin die konzentrierte Lösung aus Benazeprilhydrochlorid in Ethanol bei einer Temperatur von etwa 10 bis 35 °C, vorzugsweise 20 bis 30 °C, zu Diisopropylether zugesetzt wird.

5. Verfahren nach Anspruch 1, worin die kristalline Form von Benazeprilhydrochlorid durch Filtration isoliert wird und unter reduziertem Druck bei 45 bis 50 °C getrocknet wird.

## Revendications

1. Procédé perfectionné pour la cristallisation de chlorhydrate de bénazépril pour obtenir une pureté diastéréomérique d'au moins 99,8%, qui comprend (a) la réalisation d'une solution concentrée de chlorhydrate de bénazépril dans de l'éthanol, (b) l'addition de la solution obtenue à un éther isopropylique.

2. Procédé selon la revendication 1, où le chlorhydrate de bénazépril est obtenu sous une forme cristalline ayant un motif de diffraction de poudre caractéristique aux rayons X avec 20 valeurs à 6,7, 10,1, 12,0, 13,8, 15,1, 16,4, 17,4, 19,0, 19,6, 20,2, 20,9, 21,0, 25,3, 25,5, 26,4, 26,6, 27,6, 28,0, 31,0, 32,7.

3. Procédé selon la revendication 1, où l'éthanol est de l'éthanol absolu.

4. Procédé selon la revendication 1, où la solution concentrée de chlorhydrate de bénazépril dans de l'éthanol est ajoutée à l'éther isopropylique à une température d'environ 10-35°C, de préférence à 20-30°C.

5. Procédé selon la revendication 1, où la forme cristalline du chlorhydrate de bénazépril est isolée par filtration et séchée sous pression réduite à 45-50°C.
